# EUROPEAN PATENT APPLICATION

(11) **EP 3 949 874 A1**
(43) Date of publication of application: **09.02.2022**
(21) Application number: 20777961.2
(22) Date of filing: 20.03.2020
(51) Int. Cl.: A61B 17/12, A61B 17/122

(54) **CLIP APPLIER SYSTEM**

(30) Priority: 28.03.2019 JP 2019063568
(71) Applicant: Kawasaki Jukogyo Kabushiki Kaisha, Hyogo 650-8670 (JP)
(72) Inventor: TANAKA, Hideki, Hyogo 650-8670 (JP); OGATA, Mariko, Hyogo 650-8670 (JP); HOMMA, Toshiyuki, Hyogo 650-8670 (JP)
(74) Representative: EIP
(86) International application number: PCT/JP2020/012563
(87) International publication number: WO 2020/196350

(57) **Abstract**

A clip applier system includes: a clip applier that opens and closes a pair of jaws by supplying an operating fluid from a driving device to an opening/closing mechanism, which opens and closes the pair of jaws with a degree of opening corresponding to the amount of the operating fluid supplied to and discharged from the opening/closing mechanism, and discharging the operating fluid from the opening/closing mechanism to the driving device; an operation device that outputs an operation signal corresponding to the amount of operation performed on an operation tool; a detection device that outputs a signal corresponding to the degree of opening of the pair of the jaws or a detection value corresponding to the degree of opening of the pair of the jaws; and a control device that performs an opening degree control. In the opening degree control, the control device controls the motion of the driving device according to a difference between a command based on the operation signal from the operation device and an actual value based on the signal from the detection device.

## Description

### Technical Field

The present invention relates to clip applier systems for ligating tubular tissues or blood vessels in surgery.

### Background Art

In surgery using a surgical assist robot system, a surgical procedure is performed by inserting an instrument attached to the tip of a robot arm into a patient's body, and various instruments for various uses can be attached to the tip of the robot arm. As such a surgical assist robot system, the surgical system disclosed in Patent Literature (PTL 1) is known, for example, and examples of the instrument to be attached include a clip applier. The clip applier is medical equipment for ligating a tubular tissue or a blood vessel, and is used to ligate a tubular tissue or a blood vessel by closing a clip in a ligating position.

### Citation List

### Patent Literature

PTL 1: Japanese Laid-Open Patent Application Publication No. 2017-189495

### Summary of Invention

### Technical Problem

In a clip applier, a clip is loaded on a pair of jaws, and the clip is squeezed by closing the pair of jaws. The clip is made of resin, metal, or the like; for example, in the case where a metal clip is squeezed, the pair of jaws need to be closed with great gripping force. Therefore, the clip applier is desirably configured to be able to generate great gripping force. On the other hand, in the case where the clip applier is applied to the surgical system, the following may happen. Specifically, due to the ability to generate great gripping force, a tubular tissue or a blood vessel may be nipped and damaged, or a surgeon may fail to grasp how great the gripping force is and cause breakage by nipping the clip with greater gripping force than necessary.

Thus, the present invention has an object to provide a clip applier system capable of reducing clip breakage or damage to tubular tissues or blood vessels.

### Solution to Problem

A clip applier system according to the first invention includes: a clip applier that opens and closes a pair of jaws by supplying an operating fluid from a driving device to an opening/closing mechanism and discharging the operating fluid from the opening/closing mechanism to the driving device, the opening/closing mechanism being configured to open and close the pair of jaws with a degree of opening corresponding to an amount of the operating fluid supplied to and discharged from the opening/closing mechanism; an operation device that outputs an operation signal corresponding to an amount of operation performed on an operation tool; a detection device that outputs a signal corresponding to the degree of opening of the pair of the jaws or a detection value corresponding to the degree of opening of the pair of the jaws; and a control device that performs an opening degree control. In the opening degree control, the control device controls a motion of the driving device according to a difference between a command based on the operation signal from the operation device and an actual value based on the signal from the detection device.

According to the present invention, an amount of the operating fluid that corresponds to the amount of operation is supplied to or discharged from the opening/closing mechanism, and the pair of jaws is closed with a degree of opening corresponding to the amount of the operating fluid. Therefore, it is possible to prevent the pair of jaws from being closed over the limit and thus reduce clip breakage or damage to tubular tissues, blood vessels, or the like of patients.

In the above-described invention, it is preferable that the opening/closing mechanism operate the pair of the jaws with gripping force corresponding to a hydraulic pressure of the operating fluid supplied to and discharged from the opening/closing mechanism, the control device select and perform one of the opening degree control and a gripping force control, and in the gripping force control, the control device control the motion of the driving device to increase or decrease the hydraulic pressure of the operating fluid according to an increment or decrement of the command based on the operation signal from the operation device.

According to this configuration, the pair of jaws can be closed with the gripping force corresponding to the amount of operation, and therefore it is possible to prevent greater gripping force than necessary from acting on clips and tubular tissues, blood vessels, or the like of patients and thus reduce clip breakage or damage to the tubular tissues, the blood vessels, or the like of the patients.

A clip applier system according to the second invention includes: a clip applier that opens and closes a pair of jaws by supplying an operating fluid from a driving device to an opening/closing mechanism and discharging the operating fluid from the opening/closing mechanism to the driving device, the opening/closing mechanism being configured to open and close the pair of jaws with the operating fluid supplied to and discharged from the opening/closing mechanism and operate the pair of the jaws with gripping force corresponding to a hydraulic pressure of the operating fluid; an operation device that outputs an operation signal corresponding to an amount of operation performed on an operation tool; and a control device that performs a gripping force control. In the gripping force control, the control device controls a motion of the driving device to increase or decrease the hydraulic pressure of the operating fluid according to an increment or decrement of a command based on the operation signal from the operation device.

According to the present invention, the pair of jaws can be closed with the gripping force corresponding to the amount of operation, and therefore it is possible to prevent greater gripping force than necessary from acting on clips and tubular tissues, blood vessels, or the like of patients and thus reduce clip breakage or damage to the tubular tissues, the blood vessels, or the like of the patients.

In the above-described invention, it is preferable that the clip applier system further include a selection device capable of selecting a half-closed clip control, and when the selection device selects the half-closed clip control, the control device perform the half-closed clip control, and in the half-closed clip control, the control device control the motion of the driving device to maintain a hydraulic pressure of the operating fluid.

According to this configuration, it is possible to prevent a clip from being squeezed under unintentional, great gripping force acting on the clip when a surgeon performs a half-closed clip procedure.

In the above-described invention, it is preferable that the control device determine whether or not a clip interposed between the pair of the jaws has been loaded, and when the control device determines that the clip has not been loaded, the control device restrict a closing motion of the pair of the jaws.

According to this configuration, it is possible to protect tubular tissues, blood vessels, or the like of patients from damage due to a direct hold between the pair of jaws.

In the above-described invention, it is preferable that the control device restrict an opening motion of the pair of the jaws until a predetermined condition is satisfied, and when the predetermined condition is satisfied, the control device control the motion of the driving device to permit the opening motion of the pair of the jaws.

According to this configuration, it is possible to minimize detachment of the clip from the pair of jaws before the predetermined condition is satisfied, for example, before a tubular tissue, a blood vessel, or the like of a patient is ligated.

A clip applier system according to the third invention includes: a clip applier that opens and closes a pair of jaws by driving, using a driving device, an opening/closing mechanism that opens and closes the pair of the jaws; an operation device that outputs an operation signal corresponding to an amount of operation performed on an operation tool; a detection device that outputs a signal corresponding to a degree of opening of the pair of the jaws or a detection value corresponding to the degree of opening of the pair of the jaws; and a control device that selects and performs one of an opening degree control and a gripping force control. In the opening degree control, the control device controls a motion of the driving device according to a difference between a command based on the operation signal from the operation device and an actual value based on the signal from the detection device, and in the gripping force control, the control device controls the motion of the driving device to increase or decrease gripping force according to an increment or decrement of a command based on the operation signal from the operation device.

According to the present invention, in the opening degree control, the pair of jaws can be closed with the degree of opening corresponding to the amount of operation, and therefore it is possible to prevent the pair of jaws from being closed over the limit and thus reduce clip breakage or damage to tubular tissues, blood vessels, or the like of patients. In the gripping force control, the pair of jaws can be closed with the gripping force corresponding to the amount of operation, and therefore it is possible to prevent greater gripping force than necessary from acting on clips and tubular tissues, blood vessels, or the like of patients and thus reduce clip breakage or damage to the tubular tissues, the blood vessels, or the like of the patients.

A clip applier system according to the fourth invention includes: a clip applier that opens and closes a pair of jaws by driving, using a driving device, an opening/closing mechanism that opens and closes the pair of the jaws; an operation device that outputs an operation signal corresponding to an amount of operation performed on an operation tool; and a control device the controls a motion of the driving device to increase or decrease gripping force of the pair of the jaws according to a command based on the operation signal from the operation device. The control device performs a half-closed clip control. In the half-closed clip control, the control device controls the motion of the driving device to maintain the gripping force regardless of an increase or a decrease in the command.

According to the present invention, it is possible to prevent a clip from being squeezed under unintentional, great gripping force acting on the clip when a surgeon performs a half-closed clip procedure.

A clip applier system according to the fifth invention includes: a clip applier that opens and closes a pair of jaws by driving, using a driving device, an opening/closing mechanism that opens and closes the pair of the jaws; an operation device that outputs an operation signal corresponding to an amount of operation performed on an operation tool; and a control device the controls a motion of the driving device to open and close the pair of the jaws according to a command based on the operation signal from the operation device. The control device determines whether or not a clip interposed between the pair of the jaws has been loaded, and when the control device determines that the clip has not been loaded, the control device controls the motion of the driving device to restrict a closing motion of the pair of the jaws.

According to the present invention, it is possible to protect tubular tissues, blood vessels, or the like of patients from damage due to a direct hold between the pair of the jaws.

A clip applier system according to the sixth invention includes: a clip applier that opens and closes a pair of jaws by driving, using a driving device, an opening/closing mechanism that opens and closes the pair of the jaws; an operation device that outputs an operation signal corresponding to an amount of operation performed on an operation tool; and a control device the controls a motion of the driving device to open and close the pair of the jaws according to a command based on the operation signal from the operation device. The control device restricts an opening motion of the pair of the jaws until a predetermined condition is satisfied, and when the predetermined condition is satisfied, the control device controls the motion of the driving device to permit the opening motion of the pair of the jaws.

According to the present invention, it is possible to minimize detachment of the clip from the pair of jaws before the predetermined condition is satisfied, for example, before a tubular tissue, a blood vessel, or the like of a patient is ligated.

### Advantageous Effects of Invention

According to the present invention, it is possible to reduce clip breakage or damage to tubular tissues, blood vessels, or the like.

The above object, other objects, features, and advantages of the present invention will be made clear by the following detailed explanation of preferred embodiments with reference to the attached drawings.

### Brief Description of Drawings

Fig. 1 is a diagram illustrating the configuration of a clip applier system according to the present embodiment.
Fig. 2 is a block diagram related to a control that is performed by a control device included in the clip applier system illustrated in Fig. 1.
Fig. 3 is a flowchart related to a clip control that is performed by a control device included in the clip applier system illustrated in Fig. 1.
Fig. 4 is a flowchart related to a fixed value control process illustrated in Fig. 3.
Fig. 5 is a flowchart related to an arbitrary value control process illustrated in Fig. 3.

### Description of Embodiments

Hereinafter, a clip applier system 1 according to an embodiment of the present invention will be described with reference to the aforementioned drawings. Note that the concept of directions mentioned in the following description is used for the sake of explanation and is not intended to limit the orientations, etc., of elements according to the present invention to these directions. The clip applier system 1 described below is merely one embodiment of the present invention. Thus, the present invention is not limited to the embodiment and may be subject to addition, deletion, and alteration within the scope of the essence of the present invention.

### <Clip Applier System>

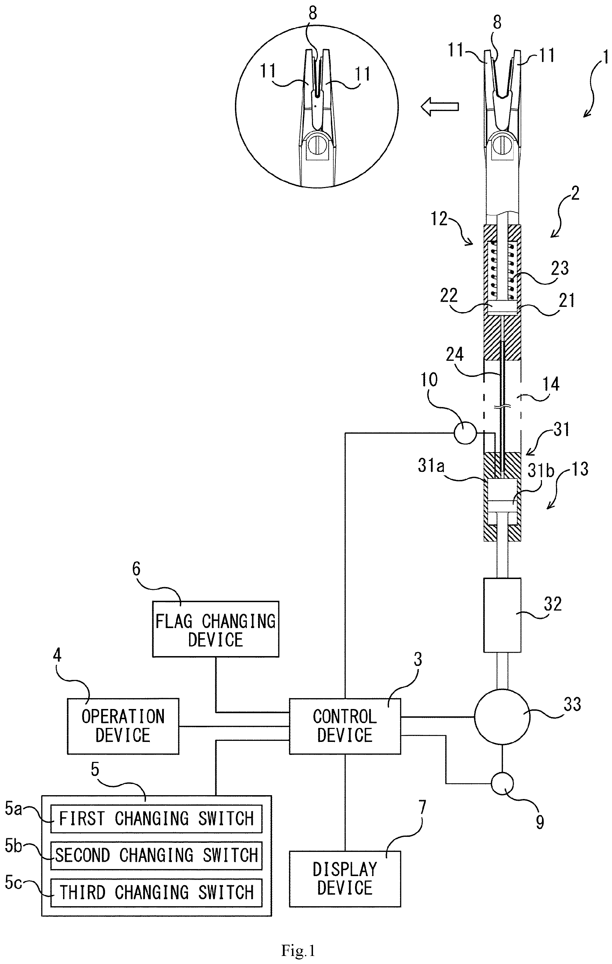

The clip applier system 1 illustrated in Fig. 1 is used in surgery using a surgical assist robot system and includes, for example, a clip applier 2. The clip applier 2 is attached to the tip of a robot arm (not illustrated in the drawings) and is used to ligate a tubular tissue, a blood vessel, or the like (hereinafter referred to as a "blood vessel, etc.") of a patient. More specifically, the clip applier 2 includes a pair of jaws 11, 11, an opening/closing mechanism 12, and a driving device 13. The pair of jaws 11, 11 is configured to be opened and closed with tip end portions thereof brought close to each other and separated from each other, and a clip 8 in V-shape can be loaded between these tip end portions. This means that the pair of jaws 11, 11 can squeeze the clip 8 by closing, and the opening/closing mechanism 12 is provided on the pair of jaws 11, 11 in order to open and close the pair of jaws 11, 11.

The opening/closing mechanism 12 includes a cylinder 21 and a piston 22, and the piston 22 is coupled to the pair of jaws 11, 11 by a link or the like. This means that the pair of jaws 11, 11 is configured to be opened and closed in conjunction with the motion of the piston 22. Note that the pair of jaws 11, 11 and the piston 22 do not necessarily need to be coupled by the link; any configuration is possible as long as the configuration allows the pair of jaws 11, 11 to be opened and closed in accordance with the extending and retracting motion of the piston 22. The piston 22 is inserted through the cylinder 21 and is extended and retracted relative to the cylinder 21 according to an operating fluid (for example, saline or oil) supplied to and discharged from the cylinder 21. Furthermore, a coil spring 23 is provided in the cylinder 21 and biases the piston 22 backward.

The opening/closing mechanism 12 configured as described above causes the piston 22 to travel according to the amount of the operating fluid supplied to and discharged from the piston 22, namely, the amount of supply and discharge, and the pair of jaws 11, 11 is opened and closed with a degree of opening corresponding to a stroke length. This means that the opening/closing mechanism 12 opens and closes the pair of jaws 11, 11 with a degree of opening corresponding to the amount of supply and discharge at the opening/closing mechanism 12. Furthermore, the opening/closing mechanism 12 is designed to operate the pair of jaws 11, 11 by a load corresponding to a hydraulic pressure received by the piston 22, and the pair of jaws 11, 11 can hold the clip 8 with gripping force corresponding to the pressure of the operating fluid. The driving device 13 is connected via a tube 24 to the opening/closing mechanism 12 having this function; the operating fluid is supplied from the driving device 13 to the cylinder 21 via the tube 24 and is discharged from the cylinder 21 to the driving device 13 via the tube 24.

The driving device 13 is configured to drive the pair of jaws 11, 11 so that the pair of jaws 11, 11 is opened and closed by supplying the operating fluid to the cylinder 21 via the tube 24 and discharging the operating fluid from the cylinder 21 via the tube 24; the driving device 13 includes a pump 31, a direct-acting mechanism 32, and a motor 33. The pump 31, which is, for example, a piston pump, is connected to the cylinder 21 via the tube 24. More specifically, the pump 31 includes a cylinder 31a and a piston 31b and can supply and discharge the operating fluid by extending and retracting the piston 31b. The pump 31 configured as just described is connected to the motor 33 via the direct-acting mechanism 32 in order to extend and retract the piston 31b.

The motor 33, which is, for example, a servomotor, generates torque corresponding to an electric current flowing thereto. Furthermore, the motor 33 is connected to the piston 31b via the direct-acting mechanism 32; the direct-acting mechanism 32 is configured as follows. Specifically, the direct-acting mechanism 32 converts a rotational motion into a linear motion; in the present embodiment, the direct-acting mechanism 32 is a ball screw mechanism. More specifically, the direct-acting mechanism 32 includes a shaft member and a slider (not illustrated in the drawings); when the shaft member is rotated in one direction using the motor 33, the slider moves along the shaft member and extends the piston 31b. When the shaft member is rotated in the other direction using the motor 33, the slider moves along the shaft member and retracts the piston 31b. In this manner, the motor 33 extends and retracts the piston 31b via the direct-acting mechanism32 and thereby supplies the operating fluid to the cylinder 21 and discharges the operating fluid from the cylinder 21 to open and close the pair of jaws 11, 11.

In the clip applier 2 configured as described above, for example, the driving device 13 is provided on a tip portion of the robot arm, and the opening/closing mechanism 12 is attached to the driving device 13 via an elongated tubular shaft 14. Therefore, by moving the robot arm, it is possible to insert the pair of jaws 11, 11 into the body of a patient and further place the pair of jaws 11, 11 in an arbitrary position on a blood vessel or the like. Furthermore, the clip applier 2 can ligate the blood vessel, etc., by positioning the blood vessel, etc., between the pair of jaws 11, 11, further closing the pair of jaws 11, 11, and squeezing the clip 8. The clip applier system 1 having this function includes a control device 3, an operation device 4, a mode changing device 5, a flag changing device 6, and a display device 7 in addition to the clip applier 2 in order to ligate the blood vessels, etc.; the control device 3 is electrically connected to the motor 33 in order to control the opening/closing motion of the pair of jaws 11, 11.

The control device 3 controls the operation of the motor 33 in accordance with various information received, and is electrically connected to the operation device 4, the mode changing device 5, and the flag changing device 6 in order to obtain the information. The operation device 4 includes, for example, an operation tool provided on a manipulator for operations, and the operation tool is configured to enable a pinching operation such as that performed to open and close scissors. Furthermore, the operation device 4 outputs, to the control device 3, an operation signal corresponding to the amount of operation performed by the pinching operation, in short, the amount of operation, and the control device 3 controls the operation of the motor 33 according to this amount of operation. Note that the operation device 4 is not limited to this configuration and may be a handle assembly including an operable trigger.

The mode changing device 5, which is one example of the selection device, includes three changing switches 5a, 5b, 5c each of which is, for example, a dial switch, a button switch, or a lever switch. Each of the switches 5a, 5b, 5c is configured to be able to change (in other words, select) a control mode by operating the switch; the control mode includes an opening degree control mode, a gripping force control mode, a half-closed clip control mode, a fixed value control mode, and an arbitrary value control mode. More specifically, the first changing switch 5a can switch between the opening degree control mode and the gripping force control mode. The second changing switch 5b can switch between ON and OFF of the half-closed clip control mode by turning ON and OFF, and the third changing switch 5c can switch between the fixed value control mode and the arbitrary value control mode. The mode changing device 5 configured as just described outputs, to the control device 3, a signal corresponding to the mode selected by the mode changing operation, and the control device 3 changes the control mode accordingly.

The flag changing device 6 is used to change, into ON, a loading completion flag indicating that the clip 8 has been loaded between the pair of jaws 11, 11; the flag changing device 6 is, for example, a button switch or a lever switch. Specifically, when a surgeon or the like operates the flag changing device 6 after the clip 8 is loaded between the pair of jaws 11, 11, the flag changing device 6 outputs a signal to the control device 3, and the control device 3 turns ON the loading completion flag. Note that in the present embodiment, the loading completion flag can be changed into ON through the operation of a surgeon, but the loading completion flag may be changed into ON using a sensor or the like that detects loading of the clip 8. Furthermore, when squeezing of the clip 8 is completed, the control device 3 turns OFF the loading completion flag. The display device 7, which is, for example, a monitor or a warning light, is further connected to the control device 3 having this function; thus, the display device 7 warns and informs the surgeon of various information.

The clip applier system 1 further includes an encoder 9 and a pressure sensor 10. The encoder 9, which is one example of the detection device, outputs a signal corresponding to the amount of rotation of the motor 33 to the control device 3, and the control device 3 obtains the amount of rotation of the motor 33. Note that the amount of rotation of the motor 33 corresponds to the position of the piston 31b and further corresponds to the degree of opening of the pair of jaws 11, 11. Therefore, the control device 3 receives, from the encoder 9, the signal corresponding to the amount of rotation that is a detection value corresponding to the degree of opening of the pair of jaws 11, 11. The pressure sensor 10 outputs a signal corresponding to a hydraulic pressure inside the cylinder 31a of the pump 31 to the control device 3, and the control device 3 obtains the pressure of the operating fluid on the basis of the signal. Note that the pressure of the operating fluid corresponds to the gripping force of the pair of jaws 11, 11.

Note that the encoder 9 is merely one example of the detection device; the detection device does not necessarily need to be the encoder 9. For example, the encoder 9 may be replaced by an angle sensor that detects the angular position of the jaws 11, 11, a stroke sensor that detects the position of the piston 22 or the piston 31b, or a distance sensor capable of detecting the amount of travel of the jaws 11, 11. This is also the case for the pressure sensor 10; the pressure sensor 10 does not necessarily need to be provided. Specifically, instead of the pressure sensor 10, a sensor that detects the hydraulic pressure in the cylinder 21 or a sensor that detects the torque of the motor 33 may be provided. Furthermore, an electric current flowing to the motor 33 may be detected; in this case, any device may be used as long as the device can detect a detection value corresponding to the gripping force of the pair of jaws 11, 11.

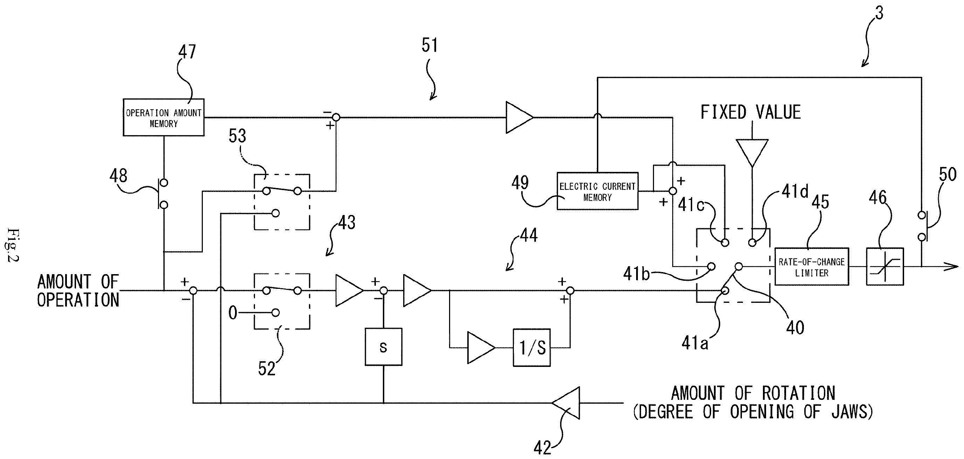

In the clip applier system 1 configured as described above, the control device 3 controls the operation of the motor 33 on the basis of the block diagram illustrated in Fig. 2; the following describes the block diagram. Specifically, the control device 3 includes a mode selection switch 40, and the mode selection switch 40 switches the terminal to be connected, to one of four terminals 41a to 41d according to the selected control mode. For example, in the case of the opening degree control mode, the mode selection switch 40 is connected to the first terminal 41a. The opening degree control mode is a mode in which the degree of opening of the pair of jaws 11, 11 is controlled according to the amount of operation performed on the operation device 4; in the present embodiment, the degree of opening of the pair of jaws 11, 11 is controlled using the amount of rotation of the motor 33.

More specifically, the control device 3 obtains the amount of operation of the operation device 4, that is, an opening degree command, and further obtains the amount of rotation of the motor 33 on the basis of the signal from the encoder 9. Moreover, using a converter 42, the control device 3 converts the amount of rotation of the motor 33 into an actual degree of opening (that is, an actual value) which is the degree of opening of the pair of jaws 11, 11. Subsequently, an opening degree control loop 43 of the control device 3 calculates a speed command on the basis of the difference between the actual degree of opening and the aforementioned opening degree command. Furthermore, the control device 3 calculates an actual speed by differentiating the actual degree of opening, and a speed control loop 44 calculates an electric current command on the basis of the actual speed and the speed command.

The electric current command calculated in this manner is limited by a rate-of-change limiter 45 so that the rate of change in the electric current command does not exceed a predetermined value, and is also limited by an electric current limiter 46 to a predetermined value or less; thus, the electric current command is output. The control device 3 provides an electric current corresponding to the output electric current command to the motor 33. Thus, the piston 31b travels to a position corresponding to the amount of operation, and the operating fluid is supplied to and discharged from the opening/closing mechanism 12 in an amount corresponding to the amount of operation. Accordingly, the pair of jaws 11, 11 is opened and closed with a degree of opening corresponding to the amount of operation of the operation device 4 (the opening degree control).

Furthermore, the control device 3 includes an operation amount memory 47, and the amount of operation of the operation device 4 is input to the operation amount memory 47 via a first control switch 48. The first control switch 48 is ON in the opening degree control mode, and the operation amount memory 47 is allowed to rewrite the received amount of operation, in other words, update the amount of operation of the operation device 4. Furthermore, the control device 3 includes an electric current command memory 49 to which the electric current command output from the electric current limiter 46 is input via a second control switch 50. The second control switch 50 is turned ON when the control mode changes into the opening degree control mode and when the control mode changes from the opening degree control mode, and the electric current command memory 49 is allowed to rewrite the received electric current command, in other words, update the electric current command. Subsequently, when the update of the electric current command in the electric current command memory 49 is completed, the second control switch 50 is turned OFF. Therefore, the electric current command memory 49 stores the electric current commands issued when the control mode changes into the opening degree control mode and when the control mode changes from the opening degree control.

Next, the gripping force control mode will be described. The gripping force control mode is a mode in which the gripping force of the pair of jaws 11, 11 is controlled according to the amount of operation performed on the operation device 4; when the control mode changes into the gripping force control mode, the mode selection switch 40 is connected to the second terminal 41b. In the gripping force control mode, the first control switch 48 is OFF, and the operation amount memory 47 stops updating the amount of operation of the operation device 4. Specifically, the operation amount memory 47 holds the amount of operation performed immediately before the change into the gripping force control mode, and a gripping force control unit 51 performs the following control on the basis of a reference amount of operation which is held in the operation amount memory 47 and an actual amount of operation which is the amount of operation of the operation device 4. Specifically, the gripping force control unit 51 calculates a difference command by multiplying the difference between the reference amount of operation and the actual amount of operation with a coefficient.

The second control switch 50 is turned ON when the terminal to be connected changes into the terminal 41b and when the terminal to be connected changes from the terminal 41b, and is turned OFF immediately after the electric current command memory 49 completes the update of the electric current command. Thus, the electric current command memory 49 stores the electric current commands issued when the terminal to be connected changes into the terminal 41b and when the terminal to be connected changes from the terminal 41b. In the gripping force control mode, the difference command calculated by the gripping force control unit 51 is added to this electric current command, meaning that the gripping force increases or decreases by the value corresponding to an increment or decrement from the reference amount of operation. Specifically, the control device 3 increases or decreases the torque of the motor 33 according to the increment or decrement of the amount of operation, and increases or decreases, according to the increment or decrement of the amount of operation, the hydraulic pressure of the operating fluid to be dispensed from the pump 31. Thus, the pair of jaws 11, 11 can be operated so as to increase or decrease the gripping force by the value corresponding to the increment or decrement from the reference amount of operation (the gripping force control).

The following describes the case where the mode selection switch 40 is connected to the fourth terminal 41d. Specifically, since a predetermined electric current command is input to the fourth terminal 41d, when the mode selection switch 40 is connected to the fourth terminal 41d, an electric current corresponding to the predetermined electric current command is provided to the motor 33. Note that the control device 3 holds two or more setting values for the predetermined electric current command to be input to the fourth terminal 41d and can select and input one of the two or more setting values according to the situation. Furthermore, the two or more setting values held in the control device 3 may be set in advance at the time of manufacture or may be set by a surgeon or the like.

Lastly, the following describes the case where the mode selection switch 40 is connected to the third terminal 41c. The electric current command memory 49 is connected to the third terminal 41c, and when the mode selection switch 40 is connected to the third terminal 41c, the electric current command held in the electric current command memory 49 is output to the third terminal 41c, and an electric current corresponding to the electric current command is provided to the motor 33. Therefore, when the mode selection switch 40 is connected to the third terminal 41c, the gripping force of the pair of jaws 11, 11 is maintained at an arbitrary value regardless of the amount of operation of the operation device 4.

Note that in the case where the mode selection switch 40 is connected to the third terminal 41c or the fourth terminal 41d, the first control switch 48 is ON, and the amount of operation of the operation device 4 stored in the operation amount memory 47 is constantly updated. Thus, even when each control mode changes into the gripping force control mode, the gripping force can be increased or decreased by the value corresponding to the increment or decrement from the reference amount of operation.

Furthermore, the control device 3 includes two anti-return switches 52, 53. The first anti-return switch 52 switches, to zero, the speed command output from the opening degree control loop 43. Thus, the degree of opening of the pair of jaws 11, 11 is maintained independently of the amount of operation of the operation device 4, and the opening and closing of the pair of jaws 11, 11 are restricted. The second anti-return switch 53 inputs an actual degree of opening (that is, the same value as the reference amount of operation), instead of the actual amount of operation, to the gripping force control unit 51. Thus, by adding the electric current command held in the electric current command memory 49 to the difference command obtained from the gripping force control unit 51, the electric current command to be input to the second terminal has a value corresponding to the actual degree of opening, allowing the degree of opening of the pair of jaws 11, 11 to be maintained at a certain value independently of the amount of operation of the operation device 4. Therefore, it is possible to restrict the opening and closing of the pair of jaws 11, 11 by operating the anti-return switches 52, 53.

The control device 3 configured as described above changes the control mode as follows according to the operation performed on each of the changing switches 5a to 5c of the mode changing device 5. For example, the control device 3 changes the control mode into one of the opening degree control mode and the gripping force control mode according to the operation performed on the first changing switch 5a. Subsequently, when the second changing switch 5b is turned ON (in other words, the half-closed clip control mode is turned ON), the control device 3 can change the control mode into the control mode selected by the third changing switch 5c (specifically, the fixed value control mode or the arbitrary value control mode).

### <Mode of Use of Clip Applier System>

The clip applier system 1 configured as described above can ligate a blood vessel, etc., by squeezing the clip 8 as described earlier, but there is a risk that the blood vessel, etc., may be damaged if the pair of jaws 11, 11 take a direct hold of the blood vessel, etc., without the clip 8 having been loaded. Therefore, the control device 3 has an interlock function to keep the pair of jaws 11, 11 from being closed without the clip 8 having been loaded. Specifically, the control device 3 determines whether the loading completion flag in the flag changing device 6 is ON or OFF, and when the loading completion flag is OFF, connects the selection switch 40 to the fourth terminal 41d. Here, the predetermined electric current command to be input to the fourth terminal 41d is set to zero. Therefore, the pair of jaws 11, 11 are kept open regardless of whether or not the operation device 4 is operated, and the opening/closing motion of the pair of jaws 11, 11 is restricted. On the other hand, when the loading completion flag in the flag changing device 6 is turned ON, the control device 3 connects the selection switch 40 to one of the terminals 41a to 41d that corresponds to the control mode, and changes the setting value of the predetermined electric current command to make the pair of jaws 11, 11 closable.

Furthermore, in the clip applier system 1, there is a possibility that when the pair of jaws 11, 11 is opened in the middle of the squeezing process, the clip 8 may fall off. Therefore, the control device 3 has an anti-opening function in order to restrict the opening motion of the pair of jaws 11, 11 in the middle of the squeezing process. Specifically, using the encoder 9, the control device 3 determines whether or not the pair of jaws 11, 11 has started the squeezing motion. For example, when the control device 3 detects, on the basis of the signal from the encoder 9, that the motor 33 is rotating forward, the control device 3 determines that the pair of jaws 11, 11 is being closed to squeeze the clip 8. The control device 3 then actuates the two anti-return switches 52, 53 when the operation device 4 is operated to open the pair of jaws 11, 11, in other words, when an opening operation is performed. Thus, it is possible to reduce the occurrence of the pair of jaws 11, 11 being opened in the middle of the squeezing process.

Furthermore, when the opening operation is followed by an operation of the operation device 4 to close the pair of jaws 11, 11, that is, a closing motion, and the amount of operation of the operation device 4 exceeds the amount of operation performed before the opening operation, the operation device 4 stops the operations of the two anti-return switches 52, 53. Thus, the degree of opening of the pair of jaws 11, 11 is controlled to be a degree of opening corresponding to the amount of operation again. Note that the anti-opening function of the control device 3 is enabled mainly when the loading completion flag is ON, and is disabled when the clip 8 is completely squeezed as described later. Specifically, when it is determined that the amount of rotation detected on the basis of the signal from the encoder 9 is greater than or equal to a predetermined amount of rotation (in other words, the degree of opening of the pair of jaws 11, 11 is less than or equal to a predetermined degree of opening) and the squeezing of the clip 8 is completed (in other words, a predetermined condition is satisfied), the control device 3 disables the anti-opening function. Thus, after the squeezing of the clip 8 is completed, that is, after ligation, the opening motion of the pair of jaws 11, 11 is permitted, and a surgeon can separate the pair of jaws 11, 11 from the blood vessel, etc.

Meanwhile, after the squeezing of the clip 8 is completed, the closing motion of the pair of jaws 11, 11 is restricted to the contrary. Specifically, in the opening motion, there are cases where the clip 8 has not been loaded between the pair of jaws 11, 11 and the blood vessel, etc., may be damaged by the pair of jaws 11, 11 taking a direct hold of the blood vessel, etc., without the clip 8 having been loaded. Therefore, when the opening motion starts, the closing motion of the pair of jaws 11, 11 is restricted. Specifically, if the closing operation is performed after the squeezing is completed, the control device 3 actuates the two anti-return switches 52, 53. Thus, the pair of jaws 11, 11 is maintained with that degree of opening, and the closing motion of the pair of jaws 11, 11 is inhibited. Furthermore, as in the case of the middle of the squeezing process, when, after returning to the closing motion, the amount of operation of the operation device 4 becomes less than the amount of operation performed before the closing operation, the operation device 4 stops the operations of the two anti-return switches 52, 53. Thus, the degree of opening of the pair of jaws 11, 11 is controlled to be a degree of opening corresponding to the amount of operation again. Note that this function is disabled when the loading completion flag is turned OFF.

With the clip applier system 1 having this function, a blood vessel, etc., is ligated as follows. For example, when the second changing switch 5b is OFF, the control device 3 performs one of the opening degree control and the gripping force control according to the control mode selected by the first changing switch 5a. Thus, the degree of opening of the pair of jaws 11, 11 is controlled to be a degree of opening corresponding to the amount of operation performed by a surgeon on the operation device 4 or the gripping force of the pair of jaws 11, 11 is controlled to be gripping force corresponding to the amount of operation.

Note that a surgeon can change the control mode by the first changing switch 5a even in the middle of the closing motion, and when the control mode is changed, the control device 3 controls the degree of opening or the gripping force as follows. For example, when the control mode is changed from the opening degree control mode into the gripping force control mode, the gripping force is increased by the value corresponding to the amount of operation performed after the change. Specifically, upon transition from the opening degree control mode to the gripping force control mode, the control device 3 holds, in the electric current command memory 49, an electric current command issued upon the transition, and holds the reference amount of operation in the operation amount memory 47. Therefore, after the transition to the gripping force control mode, the control device 3 can increase the electric current command according to the increment from the reference amount of operation, and increase the gripping force by the value corresponding to the increment from the reference amount of operation. Thus, it is possible to reduce a sharp increase in the gripping force after the transition to the gripping force control mode. On the other hand, at the time of transition of the control mode from the gripping force control mode to the opening degree control mode by the first changing switch 5a, the opening degree control is performed on the basis of the amount of operation of the operation device 4, and the degree of opening of the pair of jaws 11, 11 is controlled to be a degree of opening corresponding to the amount of operation. Therefore, after the transition, a surgeon can intuitively control the degree of opening of the pair of jaws 11, 11.

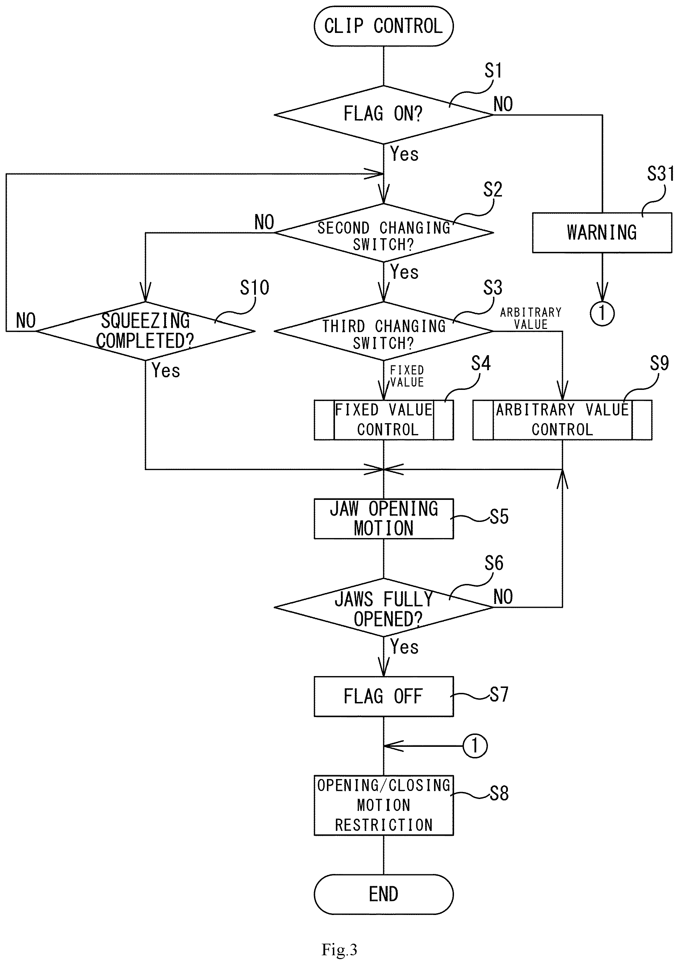

At the time of ligating a blood vessels, etc., there are cases where a technique called the half-closed clip is used. The half-closed clip is a technique in which with a blood vessel, etc., positioned between the pair of jaws 11, 11 having the clip 8 loaded thereon, the pair of jaws 11, 11 is slightly closed and moved along the blood vessel, etc., without changing the gap between the pair of jaws 11, 11. A surgeon can move the pair of jaws 11, 11 to a desired position while peeling off other parts around the blood vessel, etc., by the half-closed clip. When the pair of jaws 11, 11 reaches the desired position, the clip 8 is squeezed by the pair of jaws 11, 11 and ligates the blood vessel, etc. With reference to the flowcharts illustrated in Figs. 3 to 5, the following describes a control procedure which the control device 3 performs, including the case where the half-closed clip just described is applied. The control device 3 performs the clip control process such as that illustrated in Fig. 3 in order to ligate the blood vessel, etc., and when the clip control process is performed, the processing transitions to Step S1.

In Step S1 which is a flag determination process, the control device 3 determines whether the loading completion flag is ON or OFF. Subsequently, when it is determined that the loading completion flag is ON, the processing transitions to Step S2. In Step S2 which is a second changing switch determination step, the status of the second changing switch 5b is determined. When it is determined that the second changing switch 5b is ON, the processing transitions to Step S3 in order to perform the half-closed clip control. In Step S3 which is a control mode determination step, it is determined which of the fixed value control mode and the arbitrary value control mode has been selected by the third changing switch 5c as the control mode. When the fixed value control mode has been selected, the processing transitions to Step S4.

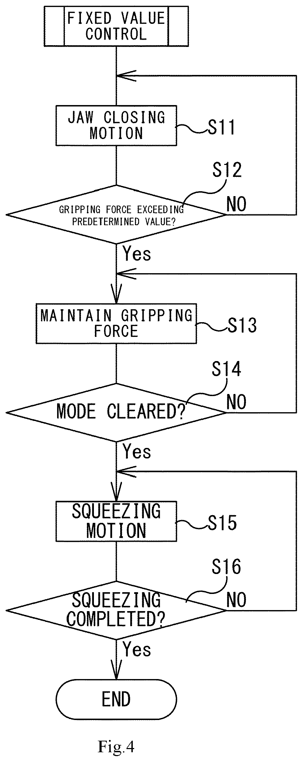

The fixed value control process in Step S4 is a process in which the gripping force applied during the half-closed clip is fixed to a predetermined value. Specifically, when the fixed value control process is performed, a process such as that illustrated in Fig. 4 is performed; the processing transitions to Step S11. In Step S11 which is a jaw closing motion step, the pair of jaws 11, 11 is closed in order to perform the half-closed clip. Specifically, the control device 3 closes the pair of jaws 11, 11 according to the amount of operation of the operation device 4. Note that at the time of closing, the degree of opening or the gripping force of the pair of jaws 11, 11 is controlled according to the control mode (the opening degree control mode or the gripping force control mode) selected by the changing switch 5a. As the pair of jaws 11, 11 is closed, the processing transitions to Step S12.

In Step S12 which is a gripping force determination step, it is determined whether or not the gripping force of the pair of jaws 11, 11 has reached a predetermined value. As mentioned earlier, the pressure of the operating fluid corresponds to the gripping force of the pair of jaws 11, 11, and whether or not the gripping force of the pair of jaws 11, 11 has reached a predetermined value is determined on the basis of the signal from the pressure sensor 10. Note that in the clip applier system 1, the approximate relationship between the degree of opening and the gripping force is predetermined for each clip 8 to be used, as mentioned earlier. Therefore, the degree of opening may be used to determine whether or not the gripping force has reached the predetermined value. When the gripping force has not reached the predetermined value (different from zero; for example, 100N), the processing returns to Step S12, and the jaw closing motion continues again. On the other hand, when the detected gripping force has reached the predetermined value, the processing transitions to Step S13.

In Step S13 which is a gripping force maintaining step, the fixed value control is performed in order to maintain the gripping force at the predetermined value. Specifically, the control device 3 connects the mode selection switch 40 to the fourth terminal 41d (refer to Fig. 2) and provides, to the motor 33, an electric current corresponding to the predetermined electric current command output to the fourth terminal 41d. Thus, the gripping force of the pair of jaws 11, 11 is maintained at the predetermined value. After the gripping force is maintained, a surgeon adjusts the position of the pair of jaws 11, 11 (that is, the ligating position) by moving an arm of a robot not illustrated in the drawings, and when the adjustment ends, the processing transitions to Step S14.

In Step S14 which is a mode clearing determination step, it is determined whether or not the half-closed clip control mode has been cleared. Specifically, according to the status of the second changing switch 5b, the control device 3 determines whether or not the half-closed clip control mode has been cleared. When the second changing switch 5b remains ON, it is determined that the half-closed clip control mode is maintained. Thus, the processing returns to Step S13, and the gripping force of the pair of jaws 11, 11 is continuously maintained at the predetermined value. On the other hand, when the second changing switch 5b is turned OFF, the control device 3 determines that the half-closed clip control mode has been cleared. Thus, the processing transitions to Step S15.

In Step S15 which is a squeezing motion step, the pair of jaws 11, 11 is closed according to the amount of operation, and thus the clip 8 is squeezed. Specifically, as in Step S11, the control device 3 closes the pair of jaws 11, 11 while controlling the degree of opening or the gripping force of the pair of jaws 11, 11 according to the control mode (the opening degree control mode or the gripping force control mode) selected by the changing switch 5a. Subsequently, as the pair of jaws 11, 11 is closed, the processing transitions to Step S16.

In Step S16 which is a squeezing determination step, it is determined whether or not the squeezing of the clip 8 has been completed. Specifically, the control device 3 detects the degree of opening of the pair of jaws 11, 11 and determines whether or not the degree of opening is less than the predetermined degree of opening. Note that in the present embodiment, whether or not the squeezing of the clip 8 is completed is determined on the basis of the amount of rotation of the motor 33, as mentioned earlier. Specifically, when the amount of rotation of the motor 33 is less than the predetermined amount of rotation (in other words, less than the predetermined degree of opening), it is determined that the squeezing of the clip 8 has not yet been completed. The processing then returns to Step S15, and the squeezing motion continues. On the other hand, when the amount of rotation of the motor 33 is greater than or equal to the predetermined amount of rotation (in other words, greater than or equal to the predetermined degree of opening), it is determined that the squeezing of the clip 8 has been completed. The fixed value control process then ends, and the processing transitions to Step S5.

In Step S5 which is a jaw opening motion step, the pair of jaws 11, 11 is opened in order to separate the pair of jaws 11, 11 from the clip 8 ligating the blood vessel, etc. Specifically, the control device 3 opens the pair of jaws 11, 11 according to the amount of operation of the operation device 4. Note that at the time of opening, the degree of opening or the gripping force of the pair of jaws 11, 11 is controlled according to the control mode (the opening degree control mode or the gripping force control mode) selected by the changing switch 5a. Subsequently, as the pair of jaws 11, 11 is opened, the processing transitions to Step S6.

In Step S6 which is a full open determination step, it is determined whether or not the pair of jaws 11, 11 is fully open. Specifically, the control device 3 detects the degree of opening of the pair of jaws 11, 11 and determines whether or not the detected degree of opening is less than an initial degree of opening. Note that in the present embodiment, whether or not the pair of jaws 11, 11 is fully open is determined according to whether or not the amount of rotation corresponding to the degree of opening is an initial amount of rotation (= 0). Note that the amount of rotation has a positive value for forward rotation and a negative value for backward rotation. The initial degree of opening is a degree of opening at which the clip 8 can be loaded. When it is determined that the pair of jaws 11, 11 is not fully open, the processing returns to Step S5, and the opening motion continues. On the other hand, when it is determined that the pair of jaws 11, 11 is fully open, the processing transitions to Step S7.

In Step S7 which is a flag OFF step, the loading completion flag is turned OFF, and when the loading completion flag is turned OFF, the processing transitions to Step S8. In Step S8 which is an opening/closing motion restriction step, the opening/closing motion of the pair of jaws 11, 11 is restricted. Specifically, in order to enable the interlock function, the control device 3 sets, to zero, the setting value of the predetermined electric current command to be input to the fourth terminal 41d, and connects the mode selection switch 40 to the fourth terminal 41d. Thus, the opening/closing motion of the pair of jaws 11, 11 is restricted independently of the amount of operation of the operation device 4, and it becomes impossible to close the pair of jaws 11, 11. When the opening/closing motion of the pair of jaws 11, 11 is restricted in this manner, the clip control ends.

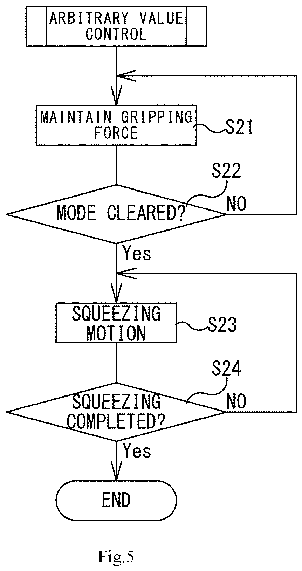

Furthermore, in the clip control process, when it is determined that the arbitrary value control mode has been selected in Step S3, the processing transitions to Step S9. The arbitrary value control process in Step S9 is a process in which the gripping force applied during the half-closed clip is arbitrarily set. Specifically, when the arbitrary value control process is performed, a process such as that illustrated in Fig. 5 is performed; the processing transitions to Step S21. In Step S21 which is a gripping force maintaining step, the arbitrary value control is performed in order to maintain the gripping force of the pair of jaws 11, 11 at an arbitrary value applied upon transition of the control mode to the arbitrary value control mode. Specifically, the control device 3 connects the mode selection switch 40 to the third terminal 41c and provides, to the motor 33, an electric current corresponding to the electric current command held in the electric current command memory 49. Therefore, the gripping force of the pair of jaws 11, 11 can be set to the arbitrary value applied upon the transition and can be maintained at said arbitrary value. After the gripping force is maintained in this manner, a surgeon adjusts the position of the pair of jaws 11, 11 (that is, the ligating position) by moving an arm of a robot not illustrated in the drawings, and when the adjustment ends, the processing transitions to Step S22.

In Step S22 which is a mode clearing determination step, it is determined whether or not the half-closed clip control mode has been cleared, as in Step S14. When it is determined that the half-closed clip control mode is maintained, the processing returns to Step S21. Thus, the gripping force of the pair of jaws 11, 11 is continuously maintained at the arbitrary value. On the other hand, when it is determined that the half-closed clip control mode has been cleared, the processing transitions to Step S23. In Step S23 which is a squeezing motion step, the pair of jaws 11, 11 is closed according to the amount of operation, and thus the clip 8 is squeezed, as in Step S15. Subsequently, as the pair of jaws 11, 11 is closed, the processing transitions to Step S24. In Step S24 which is a squeezing determination step, it is determined whether or not the clip 8 has been completely squeezed, as in Step 16. When it is determined that the squeezing has not yet been completed, the processing returns to Step S23, and the squeezing motion continues. On the other hand, when it is determined that the squeezing of the clip 8 has been completed, the arbitrary value control process ends, and the processing transitions to Step S5.

Furthermore, in the clip control process, by keeping the second changing switch 5b OFF, it is possible to close the pair of jaws 11, 11 according to the amount of operation of the operation device 4 without using the half-closed clip control mode. In this case, it is determined in Step S2 that the second changing switch 5b is OFF, and the processing transitions to Step S10. In Step S10 which is a squeezing determination step, it is determined whether or not the clip 8 has been completely squeezed, as in Step 16. When it is determined that the squeezing has not yet been completed, the processing returns to Step S2, and the determination on the status of the second changing switch 5b and the determination on the squeezing are repeated. On the other hand, when it is determined that the squeezing has been completed, the processing transitions to Step S5.

Furthermore, in the clip control process, when the control device 3 determines in Step S1 that the loading completion flag is OFF, the processing transitions to Step S31. In Step S31 which is an incomplete-loading warning step, the control device 3 issues, via the display device 7, a warning indicating that the clip 8 has not yet been loaded. When the warning is issued, the processing transitions to Step S8, the opening/closing motion is restricted, and then the clip control ends.

In the clip applier system 1 configured as described above, the pair of jaws 11, 11 can be closed with the degree of opening corresponding to the amount of operation of the operation device 4 in the opening degree control mode, and thus even when a great gripping force is generated at the pair of jaws 11, 11 to squeeze the clip 8, the pair of jaws 11, 11 can be kept from being closed over the limit and damaging blood vessels, etc., or breaking the clip 8. Furthermore, in the gripping force control mode, the pair of jaws 11, 11 can be closed with the gripping force corresponding to the amount of operation, and therefore it is possible to prevent greater gripping force than necessary from acting on blood vessels, etc., or the clip 8 and thus reduce damage to the blood vessels, etc., or breakage of the clip 8.

Furthermore, in the clip applier system 1, the gripping force of the pair of jaws 11, 11 that is applied during the half-closed clip control in the fixed value control mode can be maintained at the predetermined value. Furthermore, by switching to the arbitrary value control mode, it is possible to maintain the gripping force of the pair of jaws 11, 11 at an arbitrary value that is applied upon the mode transition. Thus, it is possible to reduce the occurrence of the clip 8 being squeezed by an intentional amount of operation at the time when a surgeon performs the half-closed clip.

Furthermore, in the clip applier system 1, since the opening motion of the pair of jaws 11, 11 is restricted before the clip 8 is completely squeezed, it is possible to reduce the occurrence of the clip 8 being separated from the pair of jaws 11, 11 at the time of ligating blood vessels, etc., during ligation. Furthermore, since the closing motion of the pair of jaws 11, 11 with no clip 8 loaded thereon is restricted, it is possible to protect blood vessels, etc., from damage due to a direct hold between the pair of the jaws 11, 11.

### Other Embodiments

In the clip applier system 1, the pair of jaws 11, 11 of the clip applier 2 is operated with the operating fluid, but the method for driving the clip applier 2 is not limited to a hydraulic driving method such as that described earlier. The method for driving the clip applier 2 may be, for example, a wire driving method. Specifically, in the wire driving method, the motor 33 and the opening/closing mechanism are connected by a wire, and the motor 33 is rotated to open and close the pair of jaws 11, 11 via the opening/closing mechanism. By controlling the operation of the motor 33 as in the case of the hydraulic driving method, a clip applier system of the wire drive type produces substantially the same advantageous effects as the clip applier system 1.

Furthermore, the clip applier system 1 is configured to be able to switch between the opening degree control mode and the gripping force control mode, but does not necessarily need to be able to switch the control mode; it is sufficient that the clip applier system 1 have any of the control modes. Moreover, the clip applier system 1 does not necessarily need to have the fixed value control mode and the arbitrary value control mode (in other words, there is no need to provide the half-closed clip control mode) and may have only one of the control modes.

From the foregoing description, many modifications and other embodiments of the present invention would be obvious to a person having ordinary skill in the art. Therefore, the foregoing description should be interpreted only as an example and is provided for the purpose of teaching the best mode for carrying out the present invention to a person having ordinary skill in the art. Substantial changes in details of the structures and/or functions of the present invention are possible within the spirit of the present invention.

### Reference Signs List

- 1: clip applier system
- 2: clip applier
- 3: control device
- 4: operation device
- 5: mode changing device
- 6: flag changing device
- 7: display device
- 8: clip
- 9: encoder
- 11, 11: jaw
- 12: opening/closing mechanism
- 13: driving device

## Claims

1. A clip applier system, comprising:
a clip applier that opens and closes a pair of jaws by supplying an operating fluid from a driving device to an opening/closing mechanism and discharging the operating fluid from the opening/closing mechanism to the driving device, the opening/closing mechanism being configured to open and close the pair of jaws with a degree of opening corresponding to an amount of the operating fluid supplied to and discharged from the opening/closing mechanism;
an operation device that outputs an operation signal corresponding to an amount of operation performed on an operation tool;
a detection device that outputs a signal corresponding to the degree of opening of the pair of the jaws or a detection value corresponding to the degree of opening of the pair of the jaws; and
a control device that performs an opening degree control, wherein
in the opening degree control, the control device controls a motion of the driving device according to a difference between a command based on the operation signal from the operation device and an actual value based on the signal from the detection device.

2. The clip applier system according to claim 1, wherein:
the opening/closing mechanism operates the pair of the jaws with gripping force corresponding to a hydraulic pressure of the operating fluid supplied to and discharged from the opening/closing mechanism;
the control device selects and performs one of the opening degree control and a gripping force control; and
in the gripping force control, the control device controls the motion of the driving device to increase or decrease the hydraulic pressure of the operating fluid according to an increment or decrement of the command based on the operation signal from the operation device.

3. A clip applier system, comprising:
a clip applier that opens and closes a pair of jaws by supplying an operating fluid from a driving device to an opening/closing mechanism and discharging the operating fluid from the opening/closing mechanism to the driving device, the opening/closing mechanism being configured to open and close the pair of jaws with the operating fluid supplied to and discharged from the opening/closing mechanism and operate the pair of the jaws with gripping force corresponding to a hydraulic pressure of the operating fluid;
an operation device that outputs an operation signal corresponding to an amount of operation performed on an operation tool; and
a control device that performs a gripping force control, wherein:
in the gripping force control, the control device controls a motion of the driving device to increase or decrease the hydraulic pressure of the operating fluid according to an increment or decrement of a command based on the operation signal from the operation device.

4. The clip applier system according to any one of claims 1 to 3, further comprising:
a selection device capable of selecting a half-closed clip control, wherein:
when the selection device selects the half-closed clip control, the control device performs the half-closed clip control; and
in the half-closed clip control, the control device controls the motion of the driving device to maintain a hydraulic pressure of the operating fluid.

5. The clip applier system according to any one of claims 1 to 4, wherein:
the control device determines whether or not a clip interposed between the pair of the jaws has been loaded, and when the control device determines that the clip has not been loaded, the control device restricts a closing motion of the pair of the jaws.

6. The clip applier system according to any one of claims 1 to 5, wherein:
the control device restricts an opening motion of the pair of the jaws until a predetermined condition is satisfied, and when the predetermined condition is satisfied, the control device controls the motion of the driving device to permit the opening motion of the pair of the jaws.

7. A clip applier system, comprising:
a clip applier that opens and closes a pair of jaws by driving, using a driving device, an opening/closing mechanism that opens and closes the pair of the jaws;
an operation device that outputs an operation signal corresponding to an amount of operation performed on an operation tool;
a detection device that outputs a signal corresponding to a degree of opening of the pair of the jaws or a detection value corresponding to the degree of opening of the pair of the jaws; and
a control device that selects and performs one of an opening degree control and a gripping force control, wherein:
in the opening degree control, the control device controls a motion of the driving device according to a difference between a command based on the operation signal from the operation device and an actual value based on the signal from the detection device; and
in the gripping force control, the control device controls the motion of the driving device to increase or decrease gripping force according to an increment or decrement of a command based on the operation signal from the operation device.

8. A clip applier system, comprising:
a clip applier that opens and closes a pair of jaws by driving, using a driving device, an opening/closing mechanism that opens and closes the pair of the jaws;
an operation device that outputs an operation signal corresponding to an amount of operation performed on an operation tool; and a control device the controls a motion of the driving device to increase or decrease gripping force of the pair of the jaws according to a command based on the operation signal from the operation device, wherein: the control device performs a half-closed clip control; and
in the half-closed clip control, the control device controls the motion of the driving device to maintain the gripping force regardless of an increase or a decrease in the command.

9. A clip applier system, comprising:
a clip applier that opens and closes a pair of jaws by driving, using a driving device, an opening/closing mechanism that opens and closes the pair of the jaws;
an operation device that outputs an operation signal corresponding to an amount of operation performed on an operation tool; and
a control device the controls a motion of the driving device to open and close the pair of the jaws according to a command based on the operation signal from the operation device, wherein:
the control device determines whether or not a clip interposed between the pair of the jaws has been loaded, and when the control device determines that the clip has not been loaded, the control device controls the motion of the driving device to restrict a closing motion of the pair of the jaws.

10. A clip applier system, comprising: a clip applier that opens and closes a pair of jaws by driving, using a driving device, an opening/closing mechanism that opens and closes the pair of the jaws;
an operation device that outputs an operation signal corresponding to an amount of operation performed on an operation tool; and
a control device the controls a motion of the driving device to open and close the pair of the jaws according to a command based on the operation signal from the operation device, wherein:
the control device restricts an opening motion of the pair of the jaws until a predetermined condition is satisfied, and when the predetermined condition is satisfied, the control device controls the motion of the driving device to permit the opening motion of the pair of the jaws.
